# EUROPÄISCHE PATENTSCHRIFT

(11) **EP 1 073 607 B1**
(45) Veröffentlichungstag und Bekanntmachung des Hinweises auf die Patenterteilung: **17.05.2006**
(21) Anmeldenummer: 99917845.2
(22) Anmeldetag: 20.03.1999
(51) Int. Cl.: C01B 15/023, C07C 50/16

(54) **VERFAHREN ZUR HERSTELLUNG VON WASSERSTOFFPEROXID UND REAKTIONSTRÄGER ZU SEINER DURCHFÜHRUNG**
METHOD FOR PRODUCING HYDROGEN PEROXIDE AND REACTION CARRIERS FOR CARRYING OUT THE METHOD
PROCEDE POUR PREPARER DU PEROXYDE D'HYDROGENE ET SUPPORT DE REACTION UTILISE POUR LE METTRE EN OEUVRE

(30) Priorität: 11.04.1998 DE 19816297
(43) Veröffentlichungstag der Anmeldung: 07.02.2001
(73) Patentinhaber: Degussa AG, 40474 Düsseldorf (DE)
(72) Erfinder: GLENNEBERG, Jürgen, D-63065 Offenbach (DE); GOOR, Gustaaf, D-63457 Hanau (DE); STAAB, Eugen, D-63768 Hösbach (DE); ANGERT, Hubert, 29195-00 Coqueiral-Arancruz, E.S. (BR)
(86) Internationale Anmeldenummer: PCT/EP1999/001972
(87) Internationale Veröffentlichungsnummer: WO 1999/052819

(56) Entgegenhaltungen:
- EP-A- 0 778 085
- DE-B- 1 106 737
- DE-B- 1 112 051
- US-A- 4 374 820
- PATENT ABSTRACTS OF JAPAN vol. 008, no. 145 (C-232), 6. Juli 1984 (1984-07-06) & JP 59 051235 A (KAWASAKI KASEI KOGYO KK), 24. März 1984 (1984-03-24) in der Anmeldung erwähnt
- PATENT ABSTRACTS OF JAPAN vol. 008, no. 015 (C-206), 21. Januar 1984 (1984-01-21) & JP 58 180452 A (KAWASAKI KASEI KOGYO KK), 21. Oktober 1983 (1983-10-21) in der Anmeldung erwähnt

## Beschreibung

Die Erfindung richtet sich auf ein Verfahren zur Herstellung von Wasserstoffperoxid nach dem Anthrachinon-Kreisprozeß. Die zu verwendende Arbeitslösung enthält als Reaktionsträger mindestens zwei unterschiedlich substituierte 2-Alkylanthrachinone und/oder die entsprechenden 2-Alkyltetrahydroanthrachinone. Die Erfindung richtet sich ferner auf einen neuen Reaktionsträger.

Beim sogenannten Anthrachinon-Kreisprozeß zur Herstellung von Wasserstoffperoxid werden als Reaktionsträger fungierende 2-Alkylanthrachinone und/oder deren kernhydrierte 2-Alkyl-α- und/oder β-Tetrahydroanthrachinone in einem organischen Lösungsmittelsystem in Gegenwart eines Hydrierkatalysators mit Wasserstoff oder einem Wasserstoff enthaltenden Gas hydriert, wobei die Reaktionsträger zumindest teilweise in die Hydrochinonform überführt werden. Die einen oder mehrere Reaktionsträger in der hydrierten oder oxidierten Form und das organische Lösungsmittelsystem enthaltende Lösung wird allgemein als Arbeitslösung bezeichnet. Nach der Hydrierstufe wird die Arbeitslösung vom Hydrierkatalysator befreit und in der Oxidationsstufe mit einem sauerstoffhaltigen Gas behandelt, wobei sich unter Bildung von Wasserstoffperoxid die Chinonform der Reaktionsträger zwrückbildet. Nach Abtrennung des gebildeten Wasserstoffperoxids von der oxidierten Arbeitslösung, üblicherweise durch Extraktion mit Wasser und/oder einer Wasserstoffperoxid enthaltenden wäßrigen Lösung, wird die Arbeitslösung wieder der Hydrierstufe zugeführt. Außer den genannten Stufen kann der Prozeß auch eine Regenerierung der Arbeitslösung umfassen, wobei im Kreisprozeß gebildete, als Reaktionsträger unwirksame Anthrachinonderivate, wie Anthrachinonepoxide, reaktiviert und/oder 2-Alkyltetrahydroanthrachinone zu den entsprechenden 2-Alkylanthrachinonderivaten dehydriert und bei Bedarf auch Verluste an Reaktionsträgern durch Zugabe der entsprechenden 2-substituierten Anthrachinone und/oder deren Tetrahydroderivaten ersetzt werden. Eine weitere Stufe richtet sich auf die Regenerierung des Katalysators, um eine hohe Aktivität aufrechtzuerhalten. Einen Überblick über den Anthrachinon-Kreisprozeß vermittelt Ullmann's Encyclopedia of Industrial Chemistry 5th ed. (1989), Vol. A13, 447-457.

An den Reaktionsträger werden hohe Anforderungen gestellt, um in großtechnischen Anlagen eine möglichst hohe Anlagenleistung mit geringstmöglicher Störanfälligkeit und möglichst niedrigem Verlust an Reaktionsträgern zu gewährleisten. Eine der Anforderungen richtet sich insbesondere auf eine möglichst hohe Löslichkeit des Reaktionsträgers im Lösungsmittelsystem, und zwar sowohl in der Chinonform als auch in der Hydrochinonform. Die Löslichkeit der Hydrochinonform ist bestimmend für das im Dauerbetrieb erhältliche maximale H₂O₂-Äquivalent (= g H₂O₂ pro l Arbeitslösung). Weitere Anforderungen betreffen die Hydrier- und Oxidationskinetik; beide Reaktionen sollen möglichst rasch verlaufen. Da durch eine Veränderung der Struktur eines Reaktionsträgers die Hydrierung und die Oxidation oft in entgegengesetzter Weise beeinflußt werden, stellt auch ein gutes Reaktionsträgersystem aus zwei oder mehr Komponenten oft nur einen Kompromiß dar. Von Wichtigkeit sind auch eine möglichst hohe chemische Stabilität des Reaktionsträgers bei der katalytischen Hydrierung, eine hohe Oxidationsstabilität gegenüber Sauerstoff und Wasserstoffperoxid, ferner eine hohe Stabilität gegenüber Säuren oder/und Alkalien, wie sie bei der Regenerierung Anwendung finden. Schließlich soll der Reaktionsträger in Wasser möglichst unlöslich, toxikologisch unbedenklich und preiswert verfügbar sein.

Gemäß GB-Patent 1 252 822 lassen sich im Anthrachinonprozeß zur Herstellung von Wasserstoffperoxid ein oder mehrere 2-Alkylanthrachinone mit 2 bis 6 Kohlenstoffatomen in der Alkylgruppe, insbesondere 2-Ethyl-, 2-tert.-Butyl- und 2-Amylanthrachinon einsetzen. Wirksam sind auch die sich in der Hydrierstufe bildenden 2-Alkyl-tetrahydroanthrachinone.

In dem zuvor zitierten GB-Patent wird kein einziger 2-Alkylanthrachinon-Reaktionsträger mit 6 C-Atomen in der Alkylgruppe beispielhaft erwähnt oder gar hervorgehoben. In den EP-A-Dokumenten 0 286 610 und 0 778 085 wird neben anderen 2-Alkylanthrachinonen und Gemischen 2-Hexenylanthrachinon als Reaktionsträger genannt. Welches der möglichen Hexenyl-Isomere gemeint ist und ob oder welche Vorteile damit erzielt werden können, lassen sich diesem EP-Dokument nicht entnehmen. Es ist zwar bekannt, daß mit wachsender Kettenlänge des Alkylsubstituenten in 2-Alkylanthrachinonen die Chinonlöslichkeit zunimmt, gleichzeitig aber, und das ist für die Praxis eher noch wichtiger, die Hydriergeschwindigkeit stark abnimmt. Damit lag es nicht nahe, die Verwendung eines 2-C₆-Alkylanthrachinons als Reaktionsträger ernsthaft in Betracht zu ziehen.

Aus der JP-A 58 180452 und JP-A 59 051235 folgt, daß 2-(4-Methyl-3-pentenyl)-1,4-dihydroanthrachinon sowie das hieraus erhältliche 2-(4-Methyl-3-pentenyl)-anthrachinon und 2-(4-Methylpentyl)-anthrachinon als Reaktionsträger zur Herstellung von Wasserstoffperoxid eingesetzt werden können. Die Herstellung der genannten Verbindungen, die Ausgangsverbindung wird durch Diels-Alder-Reaktion aus 1,4-Naphthochinon und Myrcen erhalten, läßt sich diesen Dokumenten entnehmen. Bezüglich des Einsatzes dieser Verbindungen im Anthrachinon-Kreisprozeß zur Herstellung von Wasserstoffperoxid wird lediglich erwähnt, daß die gleichen Ergebnisse wie mit bekannten 2-Alkylanthrachinonen erhalten werden können.

Die an einen guten Reaktionsträger gestellten Anforderungen werden bei der Verwendung eines einzigen 2-Alkylanthrachinons und/oder des entsprechenden in situ gebildeten 2.-Alkyltetrahydroanthrachinons in Abhängigkeit von den Betriebsbedingungen mitunter nur teilweise erfüllt. In der Fachwelt wurden daher große Anstrengungen unternommen, den Reaktionsträger durch Verwendung von mindestens zwei verschiedenen 2-Alkylanthrachinonen und/oder deren Tetrahydroderivaten zu verbessern. Vorteilen bezüglich der einen oder anderen Anforderung an ein gutes Reaktionsträgersystem stehen aber oft Nachteile bezüglich anderer Kriterien entgegen.

Gemäß der DE-AS 11 95 279 ist es möglich, die Ausbeute an Wasserstoffperoxid zu erhöhen und/oder die Entstehung von Nebenprodukten bei der Hydrierung zu minimieren, wenn anstelle eines einzigen 2-Alkylanthrachinons, wie 2-Ethyl-, 2-Isopropyl-, 2-sec.-Butyl oder 2-tert.-Butylanthrachinon, eine nahezu eutektische Mischung aus mindestens zwei 2-Alkylanthrachinonen, wie vorzugsweise 2-Ethyl und 2-sec.-Butylanthrachinone im Gewichtsverhältnis von 27 zu 73, verwendet und der Hydriergrad unter 40 % gehalten wird. Nachteilig an diesem Verfahren ist das Erfordernis, den Hydriergrad limitieren zu müssen. Ein noch schwerwiegenderer Nachteil ist die unbefriedigende Hydrierkinetik dieser eutektischen Gemische. Ähnliche Gemische aus zwei C₁- bis C₄-Alkylanthrachinonen, die im sogenannten "Anthra"- aber auch Tetrasystem vorliegen können, sind aus dem US-Patent 2,966,397 bekannt.

Im US-Patent 4,374,820 wird vorgeschlagen, ein Gemisch aus 2-tert.-Butylanthrachihon und 2-sec.-Amylanthrachinon einschließlich deren Tetrahydroverbindungen zu verwenden. Dieses System weist zwar eine gute Oxidationskinetik, aber eine unbefriedigende Hydrierkinetik auf. In den DE-Offenlegungsschriften 11 12 051 und 11 06 737 wird dagegen empfohlen, ein Gemisch aus isomeren 2-Amylanthrachinonen, insbesondere ein Gemisch aus 2-sec.-Amyl- und 2-tert.-Amylanthrachinon und deren Tetrahydroderivaten als Reaktionsträger einzusetzen. Mit derartigen Systemen kann wegen deren guter Chinon- und Hydrochinonlöslichkeit zwar ein hohes H₂O₂-Äquivalent erzielt werden, nachteilig ist jedoch auch hier die unbefriedigende Hydrierkinetik, deren Folge eine schlechte Raum-Zeit-Ausbeute ist.

Auch die Verwendung eines Reaktionsträgersystems auf der Basis von 2-Ethylanthrachinon (EAQ) und 2-Amylanthrachinon (AAQ) und deren Tetrahydroderivaten (THEAQ und THAAQ) ist bekannt - siehe EP-A 0 453 949 und Chemical Economics Handbook-SRI International June 1992 CEH Product Review Hydrogen Peroxide. Ein Reaktionsträgersystem auf dieser Basis (EAQ/THEAQ und AAQ/THAAQ) führt im Vergleich zu einem Reaktionsträgersystem auf der Basis von 2-Ethylanthrachinon und 2-Ethyl-tetrahydroanthrachinon zu einem erhöhten H₂O₂₋Äquivalent, das sich unter betrieblichen Kreislaufbedingungen auch aufrechterhalten läßt. Nachteilig an dem Reaktionsträgersystem auf der Basis von EAQ/THEAQ und AAQ/THAAQ ist seine Störanfälligkeit in der Hydrierstufe, welche sich in einer reduzierten Wasserstoffaufnahme bemerkbar macht. Bei Verwendung eines Suspensionskatalysators, wie Pd-Mohr, zwingt dieses Verhalten dazu, einen relativ hohen Umlauf an Hydrierkatalysator sicherzustellen und bei Störungen diesen noch weiter zu erhöhen; damit sinkt aber die Wirtschaftlichkeit des Prozesses.

Aufgabe der vorliegenden Erfindung ist, ein weiteres Verfahren zur Herstellung von Wasserstoffperoxid unter Verwendung einer mindestens zwei unterschiedlich substituierte 2-Alkylanthrachinone und/oder deren Tetrahydroverbindungen enthaltenden Arbeitslösung bereitzustellen, das die Nachteile der Verfahren unter Einsatz der vorbekannten 2-Alkylanthrachinon-Kombinationen, insbesondere jener auf der Basis von Ethyl- und Amylanthrachinon und deren Tetrahydroderivaten, in geringerem Umfang aufweist. Zudem sollte das zu verwendende Reaktionsträgersystem bei guter Hydrierkinetik zu einem betrieblich sicher beherrschbaren höheren H₂O₂-Äquivalent führen und weniger störanfällig sein.

Die Aufgabe wird gelöst durch ein verfahren zur Herstellung von Wasserstoffperoxid nach dem Anthrachinon-Kreisprozeß, umfassend eine Hydrierstufe, eine Oxidationsstufe und eine Stufe zur Isolierung des Wasserstoffperoxids, unter Verwendung einer mindestens zwei unterschiedlich substituierte 2-Alkylanthrachinone und/oder deren 2-Alkyltetrahydroanthrachinone enthaltenden Arbeitslösung, dadurch gekennzeichnet, daß man eine Arbeitslösung verwendet, welche (i) mindestens einen Reaktionsträger aus der Reihe 2-(4-Methyl-3-pentenyl)-anthrachinon (IHEAQ), 2-(4-Methylpentyl)-anthrachinon (IHAQ) und deren kernhydrierten Di- und Tetrahydroanthrachirionderivaten und (ii) mindestens einen Reaktionsträger aus der Reihe 2-Ethylanthrachinon (EAQ) und/oder 2-Ethyltetrahydroanthrächinon (THEAQ) enthält, wobei die Reaktionsträger gemäß (i) in einer Menge von 5 bis 95 Mol-%, bezogen auf die Summe aller Reaktionsträger, anwesend sind.

Bei den erfindungsgemäß anwesenden Reaktionsträgerkomponenten gemäß (i) handelt es sich um eine oder mehrere Verbindungen aus der Reihe 2-(4-Methyl-3-pentenyl)-anthrachinon, nachfolgend auch als 2-Isohexenylanthrachinon oder abgekürzt als IHEAQ bezeichnet, 2-(4-Methylpentyl)-anthrachinon, nachfolgend auch als Isohexylanthrachinon oder abgekürzt als IHAQ bezeichnet, 2-(4-Methyl-3-pentenyl)-1,4-dihydroanthrachinon (= 1,4-Dihydro-IHEAQ), 1,2,3,4-Tetrahydro-IHAQ (α-THIHAQ), 5,6,7,8-Tetrahydro-IHAQ (β-THIHAQ), 5,6,7,8-Tetrahydro-IHEAQ (β-THIHEAQ) und Zwischenstufen der Hydrierung von IHEAQ und IHAQ zu THIHEAQ beziehungsweise THIHAQ unter Bedingungen des Anthrachinonprozesses. Im Kreisprozeß bildet sich aus IHAQ überwiegend β-THIHAQ neben wenig α-THIHAQ - die Abkürzung THIHAQ steht für das im Prozeß gebildete Isomerengemisch. Im erfindungsgemäßen Verfahren besonders bevorzugte Reaktionsträger gemäß (i) sind IHEAQ und IHAQ sowie deren β-Tetrahydroderivate, insbesondere β-THIHAQ. Aufgrund des Anthrachinon-Kreisprozesses bilden sich beim Einsatz von IHEAQ als Komponente gemäß (i) nach längerem Betrieb in der Arbeitslösung IHAQ und THIHAQ.

IHEAQ ist mittels einer Diels-Alder-Reaktion aus 1,4-Naphthochinon und Myrcen und einer nachgeschalteten basenkatalysierten Oxidation des gebildeten 1,4,4a,9a-Tetrahydro-IHEAQ mit Luft erhältlich. IHAQ ist durch Hydrierung von IHEAQ, beispielsweise an Pt/C, erhältlich. α-THIHAQ ist gemäß DE-A 2 150 337 durch Hydrierung von 1,4-Dihydro-IHEAQ erhältlich.

2-(4-Methylpentyl)-5,6,7,8-tetrahydroanthrachinon (THIHAQ), ein bisher nicht bekannter Reaktionsträger für den Anthrachinon-Kreisprozeß, läßt sich durch Hydrierung von IHEAQ mit Raney-Nickel oder anderen Hydrierkatalysatoren, wie Pt, Pd, Rh in metallischer oder trägergebundener Form, gewinnen; sie entsteht ferner im Anthrachinon-Kreisprozeß aus IHAQ und THIHEAQ. β-THIHEAQ ist auch erhältlich durch Diels-Alder-Reaktion von Tetrahydronaphthochinon und Myrcen mit nachfolgender basenkatalysierter Oxidation.

Bei dem/den 2-Alkylanthrachinon/en gemäß (ii) handelt es sich um 2-Ethylanthrachinon und/oder dessen Tetrahydroderivate. Obligatorisch anwesend ist ein Reaktionsträger aus der Reihe 2-Ethylanthrachinon (EAQ) und/oder 2-Ethyl-tetrahydroanthrachinon (α- und β-THEAQ, wobei
β-THEAQ in der Regel weit überwiegt).

Gemäß einer bevorzugten Ausführungsform enthält die Arbeitslösung als Reaktionsträger im wesentlichen eine Kombination aus EAQ und IHAQ oder IHEAQ mit den entsprechenden Tetrahydroverbindungen THEAQ und THIHAQ und/oder THIHEAQ. Die Erfindung wird anhand dieses Systems weiter erläutert.

Es ist möglich, eine Arbeitslösung, welche als Reaktionsträger im wesentlichen EAQ und THEAQ enthält, mit 2-Isohexenylanthrachinon (IHEAQ) oder Isohexylanthrachinon (IHAQ) und/oder deren Tetrahydroderivaten aufzustocken, um damit die H₂O₂-Kapazität zu erhöhen. Üblicherweise liegt der molare Anteil der Summe der Anthrachinon- und Tetrahydroanthrachinonderivate mit einer Isohexenyl- und/oder Isohexylgruppe, also den Produkten gemäß (i), zwischen 5 und 95 %, bezogen auf die Summe aller wirksamen Reaktionsträger. Während der Phase des Aufstockens kann der molare Anteil an Produkten gemäß (i) auch unter 5 % liegen. Es ist zweckmäßig, den molaren Anteil an Anthrachinonderivaten gemäß (i) auf Werte im Bereich von 10 bis 90 %, vorzugsweise 20 bis 80 Mol-% und insbesondere 20 bis 50 %, einzustellen und dann zu halten, da die vorteilhafte Wirkung der erfindungsgemäßen Kombination, nämlich eine Erhöhung der maximalen H₂O₂-Kapazität bei gleichzeitig besserer Hydrierkinetik im Vergleich zum nächstliegenden Reaktionsträgersystem, enthaltend EAQ/THEAQ und AAQ/THAAQ, in diesem Bereich am deutlichsten ist.

Nach Zugabe von 2-Isohexenylanthrachinon (IHEAQ) zur Arbeitslösung wird die Isohexenylgruppe im Kreisprozeß zur Isohexylgruppe hydriert. Obgleich IHEAQ für sich allein wenig oxidationsstabil ist (siehe Beispiel 3) kommt es während der Oxidationsstufe im Anthrachinon-Kreisprozeß überraschenderweise zu keinem nenneswerten Abbau der Isohexenylgruppe. Der Gehalt an IHEAQ nimmt während des Kreisprozesses langsam ab, während der Gehalt an IHAQ und THIHAQ zunimmt. Zu Beginn in geringem Umfang ebenfalls gebildetes THIHEAQ sinkt im weiteren Verlauf wieder auf Werte unterhalb der Nachweisgrenze ab.

Gemäß einer bevorzugten Ausführungsform des Verfahrens wird das Verhältnis von IHAQ zu THIHAQ und EAQ zu THEAQ während des Kreisprozesses im wesentlichen konstant gehalten. Hierzu wird ein Teil der Arbeitslösung aus dem Prozeß ausgeschleust und einer bekannten dehydrierenden Regenerierstufe zugeführt, wobei die anwesenden Tetrahydroderivate dehydriert und das Anthrachinonsystem zurückgebildet wird; der so regenerierte Arbeitslösungsanteil wird wieder dem Kreisprozeß zugeführt. Zweckmäßigerweise liegen 40 bis 80 Mol-% aller Reaktionsträger in der Tetrahydroanthrachinonform vor.

Es wurde gefunden, daß mit zunehmendem Anteil an Reaktionsträgern vom Typ (i), also insbesondere IHAQ, IHEAQ und THIHAQ, bezogen auf die Summe aller Reaktionsträger, die Hydrochinonlöslichkeit und damit die maximale Produktionskapazität an Wasserstoffperoxid (g H₂O₂/l Arbeitslösung) erhöht wird; diese Kapazität übersteigt jene analoger Systeme, welche anstelle IHAQ/THIHAQ das System AAQ/THAAQ enthalten - siehe Beispiele 4.1 bis 4.13. Ferner nimmt die Kapazität mit zunehmendem Anteil an Tetrahydroanthrachinonen zu. Entgegen der bisherigen Kenntnis, wonach die Hydrierkinetik mit zunehmender Kohlenstoffzahl in der Alkylgruppe eines Reaktionsträgers schlechter wird, ist die Hydrierkinetik der erfindungsgemäß zu verwendenden Reaktionsträger gemäß (i) mit einem Isohexyl- oder Isohexenylsubstituenten überraschenderweise wesentlich besser als die Hydrierkinetik der isomeren 2-Amylanthrachinone und 2-Amyl-tetrahydroanthrachinone (AAQ/THAAQ) - siehe Beispiele 5.1 bis 5.6. Der außergewöhnliche Vorteil des erfindungsgemäßen Verfahrens liegt darin begründet, daß mit der erfindungsgemäßen Reaktionsträgerkombination im Vergleich zum nächstliegenden vorbekannten Reaktionsträgersystem (EAQ, AAQ und deren Tetrahydroderivate) eine höhere H₂O₂-Kapazität bei gleichzeitig verbesserter Hydrierkinetik erreicht wird.

Ein weiterer Gegenstand der Erfindung richtet sich auf eine Arbeitslösung zur Herstellung von Wasserstoffperoxid, gekennzeichnet durch einen Gehalt an (i) 2-(4-Methylpentyl)-anthrachinon (IHAQ) und seinem Tetrahydroderivat 2-(4-Methylpentyl)-β-tetrahydroanthrachinon (THIHAQ) und (ii) einem Reaktionsträger aus der Reihe 2-Ethylanthrachinon und/oder dem entsprechenden 2-Ethyl-tetrahydroanthrachinon, wobei die Reaktionsträger gemäß (i) (=IHAQ und THIAHQ) in einer Menge von 5 bis 95 Mol-%, bezogen auf die Summe aller Reaktionsträger, anwesend sind und wobei der Anteil an Tetrahydroanthrachinonen, bezogen auf die Summe aller Reaktionsträger, im Bereich von 5 bis 95 Mol-% liegt.

Das erfindungsgemäße Reaktionsträgersystem kann in beliebigen gattungsgemäßen Verfahren zur Herstellung von Wasserstoffperoxid eingesetzt werden. In der Hydrierstufe können bekannte Katalysatoren, wie insbesondere solche auf der Basis von Edelmetallen, wie Pd, Pt, Ir, Rh, Ru oder Gemischen solcher Edelmetalle; und Raney-Katalysatoren von Ni, Co öder Fe verwendet werden. Die Katalysatoren können als Suspensionskatalysatoren - beispielsweise Pd-Mohr oder trägergebundene Edelmetalle - oder in Form von Festbettkatalysatoren eingesetzt werden. Bei den Trägersuspensions- und Festbettkatalysatoren handelt es sich insbesondere um Edelmetalle auf einem anörganischen Träger, wie SiO₂, TiO₂, Zeolith, BaSO₄, Polysiloxan. Schließlich kann der Katalysator auch auf der Oberfläche eines monolithischen keramischen Trägers oder eines wabenförmigen Bauteils mit ausreichend großer Oberfläche angeordnet sein. Gängige Hydrierreaktören sind als Schlaufenreaktor, Festbettreaktor, Mammutpumpenreaktor sowie als Reaktor mit integrierten Static-Mixern ausgebildet.

Die Hydrierung wird im allgemeinen bei einer Temperatur im Bereich von Raumtemperatur bis 100 °C, insbesondere 45 bis 70 °C, ausgeführt. Der Hydrierdruck liegt üblicherweise im Bereich von etwa 100 kPa bis 1 MPa, insbesondere 200 kPa bis 500 kPa. Üblicherweise wird die Hydrierung so betrieben, daß der in den Hydrierkreislauf eingebrachte Wasserstoff vollständig verbraucht und der Hydriergrad im Bereich von 30 bis 80 % gehalten wird.

Die das erfindungsgemäße Reaktionsträgersystem enthaltende Arbeitslösung enthält im allgemeinen zwei oder mehr Lösungsmittel, um die Reaktionsträgerkomponenten in der Chinonform und Hydrochinonform in Lösung zu halten. Es kommen jene Lösungsmittel und Lösungsmittelkombinationen infrage, die aus vorbekannten Anthrachinon-Kreisprozessen bekannt sind. Besonders geeignet sind Lösungsmittelkombinationen, welche neben einem Aromatenbenzin (mehrfach alkyliertes Benzol) ein oder mehrere Lösungsmittel aus der Reihe sekundärer Alkohole, wie Diisobutylcarbinol, Ester, wie Methylcyclohexylacetat, Phosphorsäureester, wie Tris(2-ethylhexyl)-phosphat, tri- und tetraalkylierter Harnstoffe, wie Tetrabutylharnstoff, cyclischer Harnstoffe, Pyrrolidone, Carbamate und N-alkylierter Caprolactame, wie N-Hexylcaprolactam, enthalten.

Wesentliche Vorteile des erfindungsgemäßen Verfahrens sind: eine gegenüber dem nächstliegenden vorbekannten Verfahren um mindestens 0,6 g H₂O₂/l Arbeitslösung höhere Kapazität; eine verbesserte Hydrierkinetik; eine geringere Störanfälligkeit bei kontinuierlichem Betrieb; eine geringere Menge umlaufendes Palladium bei Verwendung von Pd-Mohr als Katalysator.

Die Erfindung wird anhand der nachfolgenden Beispiele und Vergleichsbeispiele weiter erörtert.

### Beispiel 1

### Herstellung von 2-(4-Methyl-3-pentenyl)-anthrachinon (= 2-Isohexenyl-anthrachinon oder abgekürzt IHEAQ):

Die Herstellung erfolgte analog JP-A 59-51235 durch eine Diels-Alder-Reaktion mit nachgeschalteter Aromatisierung.

397 g (2,56 Mol) Myrcen (88 %ig, Firma Aldrich) wurden vorgelegt, anschließend wurden 405 g (2,48 Mol) 1,4-Naphthochinon (97 %ig) hinzugegeben. Die Suspension wurde 2 h bei 100 °C gerührt (die exotherme Umsetzung war bereits nach etwa 0,5 h nahezu vollständig). Das Reaktionsgemisch, ein braunes Öl, wurde in eine ethanolische Natronlauge (3 l Ethanol und 40 g NaOH) eingetragen. Die Suspension wurde bei 50 °C unter Einleiten von Luft 2 h gerührt - zunächst ging Ungelöstes in Lösung, schließlich begann ein rötlichgelber Niederschlag auszufallen. Nach dem Abkühlen wurde der Feststoff abgesaugt und mit 250 ml eisgekühltem Ethanol gewaschen. Nach dem Trocknen wurden 616 g gelbes Pulver erhalten. Die HPLC-Analyse zeigte einen Anteil von 98,5 Flächen-% IHEAQ. Das 1H-NMR-Spektrum und der Schmelzpunkt (89-90 °C, einmal aus n-Heptan umkristallisiert) entsprachen IHEAQ.

### Beispiel 2

### Herstellung von 2-(4-Methylpentyl)-β-tetrahydroanthrachinon (= β-THIHAQ).

500 g (= 1,7 Mol) IHEAQ (Rohprodukt), gelöst in 3,5 l n-Butylacetat, wurden in einem 5 l-Hydriergefäß mit Begasungsrührer bei 50 °C vorgelegt. Nach Spülen der Apparatur mit Stickstoff wurden 100 g Raney-Nickel (in 500 ml Isopropanol suspendiert) eingetragen, dann die Hydrierung gestartet. Nach 35 l H₂-Aufnahme (Hydrochinonbildung) verlangsamte sich die H₂-Aufnahme schlagartig. Nach 30 h, als 88 l H₂ aufgenommen waren, wurde die Reaktion abgebrochen. Laut HPLC enthielt das vom Katalysator und Lösungsmittel befreite Reaktionsgemisch in Flächen-%: 33 % Edukt (= IHEAQ), 45 % 2-(4-Methyl-3-pentenyl)-β-tetrahydroanthrachinon (= β-THIHEAQ), 11 % 2-(4-Methylpentyl)-anthrachinon (= IHAQ) und 8 % des gewünschten THIHAQ. Zur Entfernung von mit dem eingesetzten IHEAQ mitgeschleppten Alkaliverbindungen wurde das vom Katalysator befreite Reaktionsgemisch mit 10 %iger Salzsäure, mit wäßriger Natriumhydrogencarbonatlösung und dann mit Wasser gewaschen und getrocknet. 437 g des nach dem Entfernen des Lösungsmittels verbliebenen Rückstands wurden erneut in n-Butylacetat (3 l) Isopropanol (0,5 l) in Gegenwart von 100 g Raney-Nickel hydriert. Nach 27,5 h, als die Reaktionsmischung 74 l H₂ aufgenommen hatte, wurde die Reaktion beendet. Nach Abfiltration des Katalysators wurde das Reaktionsgemisch durch Begasen mit Luft durchoxidiert, anschließend aufkonzentriert. Der Niederschlag wurde abgesaugt und mit Isopropanol nachgewaschen und schließlich umkristallisiert. Erhalten wurden 304 g β-THIHAQ als hellgelbes Pulver: Laut HPLC betrug die Reinheit 99,7 Flächen-%. Das 1H-NMR-Spektrum entsprach β-THIHAQ.

### Beispiel 3

Bestimmung der Oxidationsstabilität von 2-(4-Methyl-3-pentenyl)anthrachinon (IHEAQ) (= Beispiel 3a) im Vergleich zu 2-Ethylanthrachinon (EAQ) (= Beispiel 3b): 0,04 Mol des Chinons IHEAQ beziehungsweise EAQ wurden in 100 ml 1,2-Dichlorbenzol gelöst. Nach Zugabe von 10 mg Azo-bisisobutyronitril als Radikalstarter wurde unter Sauerstoffüberlagerung bei 150 °C mit einem Begasungsrührer gerührt. Nach 24 h wurde der Chinongehalt chromatographisch bestimmt. Der Rest-Chinongehalt betrug bei IHEAQ 41 %, bei EAQ 90 %.

Im Gegensatz zu Beispiel 3a wird unter den Bedingungen des Anthrachinon-Kreisprozesses zur Herstellung von Wasserstoffperoxid IHEAQ überraschenderweise nicht mehr oxidativ abgebaut als EAQ, obgleich im Kreisprozeß IHEAQ lange nachweisbar ist, da es nur langsam zu 2-(4-Methylpentyl)-anthrachinon (IHAQ) hydriert wird.

### Beispiel 4

Bestimmung der Hydrochinonlöslichkeit verschiedener Reaktionsträgergemische in verschiedenen Lösungsmittelsystemen. Die eingesetzten Reaktionsträger und deren Mengen sind den Tabellen 1 und 2 zu entnehmen.

Bestimmungsmethode: Eine Suspension aus der entsprechenden Arbeitslösung und einer geringen Menge frisch gefälltem Palladium-Mohr wurde in ein magnetisch gerührtes, thermostatisiertes Doppelmantelgefäß, das mit einer Vorrichtung zur elektronischen Trübungsmessung ausgerüstet war, gefüllt. Unter Verwendung einer Gasbürette wurde langsam hydriert, zudem wurden zwecks Vermeidung einer Überhydrierung beim Annähern an die Löslichkeitsgrenze Kristallkeime in Form des Hydrochinons zugegeben. Die maximale Hydrochinonlöslichkeit war dann erreicht, wenn vom Meßgerät eine bleibende Trübung registriert wurde. Der zu diesem Zeitpunkt aufgenommene Wasserstoff wurde in H₂O₂₋Äquivalente, definiert als g H₂O₂ pro l Arbeitslösung bei 20 °C, umgerechnet.

Die Beispiele 4.1, 4.6, 4.8, 4.9 und 4.12 sind nichterfindungsgemäße Systeme mit den Reaktionsträgern EAQ/THEAQ plus AAQ/THAAQ. Erfindungsgemäße Beispiele (4.2 bis 4.5, 4.7, 4.10, 4.11 und 4.13) enthalten die Reaktionsträger EAQ/THEAQ plus IHAQ/THIHAQ oder IHEAQ/THIHAQ. Hierin bedeuten: EAQ = 2-Ethylanthrachinon, THEAQ = Tetrahydro-EAQ; AAQ = 2-Amylanthrachinon, wobei Amyl für ein Gemisch aus 1,2-Dimethylpropyl und 1,1-Dimethylpropyl (= iso-sek.- und tert.-Amyl) steht; IHAQ = 2-Isohexylanthrachinon, THIHAQ = β-Tetrahydro-IHAQ. Im Beispiel 4.7 wurde IHEAQ anstelle von IHAQ eingesetzt.

Während die Hydriertemperatur in den Beispielen 4.1 und 4.2 60 °C betrug, wurden alle übrigen Beispiele bei 50 °C durchgeführt.

In den Beispielen 4.1 bis 4.5 wurde als Lösungsmittel ein Gemisch aus C₉/C₁₀-Aromatenbenzin (AB) und Diisobutylcarbinol (DIBC) im Volumenverhältnis 6:4, in den Beispielen 4.6 und 4.7 ein Gemisch aus im wesentlichen C₉/C₁₀-Aromatenbenzin und Tetrabutylharnstoff (TBH) im Volumenverhältnis 2,5:1 eingesetzt.

In den Beispielen 4.8 bis 4.11 bestand das Lösungsmittelsystem aus C₉/C₁₀-Aromatenbenzin und Tris(2-ethylhexyl)-phosphat (TOP) im Volumenverhältnis 3:1, in den Beispielen 4.12 und 4.13 aus im wesentlichen dem C₉/C₁₀₋Aromatenbenzin und Tetrabutylharnstoff im Volumenverhältnis 2,5:1. Die Arbeitslösungen der Beispiele 4.8 bis 4.13 enthielten aufgrund deren längerer Verwendung in einer kontinuierlichen Laboranlage inerte Abbauprodukte der eingesetzten Anthrachinone. Die Arbeitslösungen der Beispiele 4.12 und 4.13 enthielten herstellungsbedingt - Einsatz einer auf EAQ/THEAQ basierenden betrieblichen Arbeitslösung - einen wesentlich größeren Anteil an Inerten als die Beispiele 4.8 bis 4.11.

**Tabelle 1:**

| Beispiel Nr. | 4.1 | 4.2 | 4.3 | 4.4 | 4.5 | 4.6 | 4.7 |
|---|---|---|---|---|---|---|---|
| Q gesamt (mol/l) | 0,70 | 0,70 | 0,70 | 0,70 | 0,70 | 0,70 | 0,70 |
| EAQ (mol/l) | 0,14 | 0,14 | 0,17 | 0,09 | 0,11 | 0,22 | 0,22 |
| THEAQ (mol/l) | 0,21 | 0,21 | 0,25 | 0,26 | 0,17 | 0,34 | 0,35 |
| AAQ (mol/l) | 0,14 | - | - | - | - | 0,06 | - |
| THAAQ (mol/l) | 0,21 | - | - | - | - | 0,08 | - |
| IHAQ *) (mol/l) | - | 0,14 | 0,11 | 0,09 | 0,17 | - | 0,06 **) |
| THIHAQ (mol/l) | - | 0,21 | 0,17 | 0,26 | 0,25 | - | 0,08 |
| H₂O₂-Äquivalent (g H₂O₂/l) | 14,3 | 14,8 | 11,9 | 12,4 | 13,0 | 11,7 | 13,7 |

| | | | | | | | |
|---|---|---|---|---|---|---|---|
| *) das eingesetzte IHAQ enthielt noch einige % IHEAQ | | | | | | | |
| **) eingesetzt wurde anstelle IHAQ reines IHEAQ | | | | | | | |

**Tabelle 2:**

| Beispiel Nr. | 4.8 | 4.9 | 4.10 | 4.11 | 4.12 | 4.13 |
|---|---|---|---|---|---|---|
| Q gesamt (mol/l) | 0,67 | 0,64 | 0,64 | 0,66 | 0,75 | 0,81 |
| EAQ (mol/l) | 0,12 | 0,12 | 0,12 | 0,11 | 0,20 | 0,21 |
| THEAQ (mol/l) | 0,29 | 0,26 | 0,29 | 0,28 | 0,38 | 0,41 |
| AAQ (mol/l) | 0,13 | 0,09 | - | - | 0,08 | - |
| THAAQ (mol/l) | 0,13 | 0,17 | - | - | 0,09 | - |
| IHAQ *) (mol/l) | - | - | 0,13 | 0,08 | - | 0,10 |
| THIHAQ (mol/l) | - | - | 0,11 | 0,19 | - | 0,09 |
| H₂O₂-Aquivalent (g H₂O₂/l) | 11,1 | 11,9 | 12,4 | 12,9 | 12,8 | 14,9 |

| | | | | | | |
|---|---|---|---|---|---|---|
| *) das eingesetzte IHAQ enthielt noch einige % IHEAQ | | | | | | |
| **) eingesetzt wurde anstelle IHAQ reines IHEAQ | | | | | | |

Aus den vergleichenden Beispielen 4.1 mit 4.2, 4.6 mit 4.7, 4.8 mit 4.10, 4.9 mit 4.11 und 4.12 mit 4.13 folgt, daß die IHAQ/THIHAQ beziehungsweise IHEAQ/THIHAQ enthaltenden Arbeitslösungen überraschenderweise zu deutlich höheren H₂O₂-Äquivalenten führen als die AAQ/THAAQ enthaltenden analogen Arbeitslösungen. Aus den Beispielen 4.3 bis 4.5 folgt, daß mit zunehmendem molaren Verhältnis der Summe der isohexyl-substituierten Anthrachinone zur Summe der ethylsubstituierten Anthrachinone das H₂O₂-Äquivalent zunimmt - das molare Verhältnis steigt von 40:60 über 50:50 zu 60:40 an, das H₂O₂-Äquivalent von 11,9 über 12,4 auf 13,0 g H₂O₂/l.

### Beispiel 5

Untersucht wurde die Hydrierkinetik unterschiedlicher Arbeitslösungen. Die Zusammensetzung der eingesetzten Anthrachinone und der Lösungsmittel sowie der Geschwindigkeitskonstanten k (mol / l ˙ min) bei einem H₂O₂₋Äquivalent von 10,0 und 12,0 g H₂O₂/l folgen aus der Tabelle.

Hydrierkinetik-Standardtest: 100 ml der Arbeitslösung und 30 mg Pd-Mohr wurden mittels Ultraschall dispergiert und in einem Doppelmantelgefäß, das mit Strombrechern und einem Begasungsrührer ausgerüstet war, bei 2000 Upm, 50 °C und einem Absolutdruck von 0,1 MPa Wasserstoff hydriert. Die Wasserstoffaufnahme (Nml) über die Zeit wurde registriert. Aus den differentiellen H₂-Aufnahmen wurden die Geschwindigkeitskonstanten k (mol / l * min) der Hydrierung in Abhängigkeit vom Umsatz berechnet. Die Hydrierkinetiken wurden bei 0,29 mol Umsatz, entsprechend einem H₂O₂₋Äquivalent der Arbeitslösung von 10,0 g H₂O₂/l und bei 0,35 mol Umsatz, entsprechend einem H₂O₂-Äquivalent von 12,0 g H₂O₂/l miteinander verglichen. Je höher k, desto schneller verläuft die Hydrierung.

**Tabelle 3:**

| Beispiel Nr. | 5.1 | 5.2 | 5.3 *) | 5.4 *) | 5.5 **) | 5.6 **) |
|---|---|---|---|---|---|---|
| Lösungsmittel | AB/DIBC (60:40) | | AB/TOP (3:1) | | AB/TOP (3:1) | |
| EAQ (mol/l) | 0,14 | 0,14 | 0,11 | 0,11 | - | - |
| THEAQ (mol/l) | 0,21 | 0,21 | 0,26 | 0,29 | 0,21 | 0,21 |
| AAQ (mol/l) | 0,14 | - | 0,13 | - | - | - |
| THAAQ (mol/l) | 0,21 | - | 0,12 | - | 0,21 | - |
| IHAQ (mol/l) | - | 0,14 | - | 0,13 | | |
| THIHAQ (mol/l) | - | 0,21 | - | 0,12 | - | 0,21 |
| k˙10⁴ bei 10,0 g H₂O₂/l | 115 | 121 | 98 | 106 | 310 | 370 |
| k˙10⁴ bei 12,0 g H₂O₂/l | 86 | 101 | 60 | 75 | 150 | 240 |

| | | | | | | |
|---|---|---|---|---|---|---|
| *) Die Arbeitslösungen der Beispiele 5.3 und 5.4 stammten aus einer mehrere Monate betriebenen Labor-Versuchsanlage und enthielten demgemäß zusätzlich inerte Bestandteile aus dem Reaktionsträger. | | | | | | |
| **) In der Messung wurde ein Pd mit höherer Aktivität eingesetzt als in den Beispielen 5.1 bis 5.4. | | | | | | |

Die vergleichenden Versuche (5.1, 5.3 und 5.5 sind nichterfindungsgemäß) zeigen, daß erfindungsgemäße Reaktionsträgersysteme eine raschere Hydrierung erlauben als vorbekannte Systeme. Die Kombination EAQ/THEAQ mit IHAQ/THIHAQ hydriert rascher als die Kombination EAQ/THEAQ mit AAQ/THAAQ (vergleiche Beispiele 5.2 mit 5.1) - dieser Unterschied wird besonders deutlich bei einem H₂O₂₋Äquivalent von 12 g/l.

### Beispiel 6

In einer Versuchsanlage für den Kreisprozeß des Anthrachinonverfahrens zur Herstellung von Wasserstoffperoxid, bestehend aus den Verfahrensstufen Hydrierung, Oxidation, Extraktion sowie Trocknung, Regenerierung und Reinigung, wurde eine Arbeitslösung, bestehend aus 75 Vol.-% Aromatenbenzin (C₉/C₁₀₋Alkylaromatengemisch), 25 Vol.-% Tris(2-ethylhexylphosphat), 0,11 mol/l 2-Ethylanthrachinon, 0,29 mol/l 2-Ethyl-tetrahydroanthrachinon, 0,13 mol/l 2-Isohexylanthrachinon und 0,12 mol/l 2-Isohexyl-tetrahydroanthrachinon, im Hinblick auf die im Dauerbetrieb erhältliche maximale H₂O₂-Produktionskapazität (g produziertes H₂O₂ pro l Arbeitslösung) untersucht. Die Hydrierstufe (Schlaufenreaktor) wurde bei einem Wasserstoffdruck von 0,35 MPa und einer Temperatur von 58 °C betrieben. Als Hydrierkatalysator wurde Pd-Mohr (0,5 bis 1 g/l) eingesetzt. Das H₂O₂-Äquivalent in der Hydrierung wurde sukzessive bis auf einen Wert von 13,0 g/l angehoben und über mehrere Tage konstant gehalten, ohne daß ein Auskristallisieren von Hydrochinonen zu beobachten war. Beim Versuch, die Kapazität auf 13,5 g/l anzuheben, kristallisierte Hydrochinon aus. Die maximale H₂O₂₋Kapazität dieser Arbeitslösung liegt demnach zwischen 13,0 und 13,5 g/l.

### Beispiel 7 (nicht erfindungsgemäß)

Analog Beispiel 6 wurde die maximale H₂O₂-Kapazit einer Arbeitslösung, bestehend aus 75 Vol.-% Aromatenbenzin (C₉/C₁₀-Alkylaromatengemisch), 25 Vol.-% Tris(2-ethylhexyl)-phosphat, 0,12 mol/l 2-Ethylanthrachinon, 0,28 mol/l 2-Ethyl-tetrahydroanthrachinon, 0,13 mol/l 2-Amylanthrachinon und 0,12 mol/l 2-Amyltetrahydroanthrachinon, ermittelt. Im Vergleich zu Beispiel 6 war hier eine deutlich höhere Menge an Pd-Mohr, nämlich 2 bis 3 g/l, zur Aufrechterhaltung der Hydrierung im Sinne einer kompletten Umsetzung des eingesetzten Wasserstoffs, notwendig. Die maximale H₂O₂-Kapazität dieser Arbeitslöung lag unter 12,4 g H₂O₂/l. Eine Steigerung des H₂O₂₋Äquivalents über 12,4 führte zu einer Ausfällung der Hydrochinone.

### Beispiel 8

Analog Beispiel 6 wurde die maximale H₂O₂-Kapazität einer Arbeitslösung, deren Lösungsmittelsystem im wesentlichen auf einem C₉/C₁₀-Aromatenbenzin und Tetrabutylharnstoff (Volumenverhältnis AB:TBH ca. 3:1) basierte, bei einer Hydriertemperatur von 60 °C, ermittelt. Die Arbeitslösung enthielt als Reaktionsträger 0,20 mol/l 2-Ethylanthrachinon, 0,35 mol/l 2-Ethyltetrahydroanthrachinon, 0,09 mol/l 2-Isohexylanthrachinon (IHAQ), das noch etwas 2-Isohexenylanthrachinon (IHEAQ) enthielt, und 0,07 mol/l 2-Isohexyl-tetrahydroanthrachinon (THIHAQ). Die Katalysatormenge betrug 0,5 bis 1,0 g/l. Die maximale H₂O₂-Kapazität lag bei mindestens 14 g H₂O₂/l. Eine Steigerung war nur deshalb nicht möglich, weil die Versuchsanlage keine höhere H₂-Begasung gestattete.

## Patentansprüche

1. Verfahren zur Herstellung von Wasserstoffperoxid nach dem Anthrachinon-Kreisprozeß, umfassend eine Hydrierstufe, eine Oxidationsstufe und eine Stufe zur Isolierung des Wasserstoffperoxids, unter Verwendung einer mindestens zwei unterschiedlich substituierte 2-Alkylanthrachinone und/oder deren 2-Alkyltetrahydroanthrachinone enthaltenden Arbeitslösung,
**dadurch gekennzeichnet,**
**daß** man eine Arbeitslösung verwendet, welche (i) mindestens einen Reaktionsträger aus der Reihe 2-(4-Methyl-3-pentenyl)-anthrachinon (IHEAQ), 2-(4-Methylpentyl) -anthrachinon (IHAQ) und deren kernhydrierten Di- und Tetrahydroanthrachinonderivaten und (ii) mindestens einen Reaktionsträger aus der Reihe 2-Ethylanthrachinon (EAQ) und/oder 2-Ethyltetrahydroanthrachinon (THEAQ) enthält, wobei die Reaktionsträger gemäß, (i) in einer Menge von 5 bis 95 Mol-%, bezogen auf die Summe aller Reaktionsträger, anwesend sind.

2. Verfahren nach Anspruch.1,
**dadurch gekennzeichnet,**
**daß** die Arbeitslösung als Reaktionsträger gemäß (i) IHAQ und/oder IHEAQ und/oder deren kernhydrierte Tetrahydroanthrachinonderivate, insbesondere β-THIBAQ, enthält.

3. Verfahren nach Anspruch 1 oder 2.
**dadurch gekennzeichnet,**
**daß** man eine Arbeitslösung verwendet, die Reaktionsträger gemäß (i) in einer Menge von 10 bis 90 Mol-%, bezogen auf die Summe an Reaktionsträgern, enthält.

4. Verfahren nach Anspruch 3,
**dadurch gekennzeichnet,**
**daß** die Arbeitslösung Reaktionsträger gemäß (i) in einer Menge von 20 bis 50 Mol-%, bezogen auf die Summe an Reaktionsträgern, enthält.

5. Verfahren nach einem der Ansprüche 1 bis 4,
**dadurch gekennzeichnet,**
**daß** man eine Arbeitslösung verwendet, welche durch Aufstocken einer mindestens einen Reaktionsträger gemäß (ii), insbesondere EAQ und THEAQ, enthaltenden Arbeitslösung während des Kreisprozesses mit 2-(4-Methyl-3-pentenyl)-anthrachinon (IHEAQ), 2-(4-Methylpentyl)-anthrachinon (IHAQ), deren Tetrahydroderivaten oder Gemischen dieser Reaktionsträger erhalten wurde.

6. Verfahren nach einem der Ansprüche 1 bis 5,
**dadurch gekennzeichnet,**
**daß** man während des Kreisprozesses eine Arbeitslösung verwendet, welche die Reaktionsträger zu 95 bis 5 %, bevorzugt 60 bis 20 %, in der Anthrachinonform und zu 5 bis 95 %, bevorzugt 40 bis 80 %, in der Tetrahydroanthrachinonform enthält.

7. Verfahren nach einem der Ansprüche 1 bis 6,
**dadurch gekennzeichnet,**
**daß** man in der Hydrierstufe als Hydrierkatalysator einen Suspensions-Edelmetallkatalysator, insbesondere Palladium-Mohr, oder einen trägergebundenen Suspensions-Edelmetallkatalysator verwendet.

8. Arbeitslösung zur Herstellung von Wasserstoffperoxid, **gekennzeichnet durch** einen Gehalt an (i) 2-(4-Methylpentyl)-anthrachinon (IHAQ) und seinem Tetrahydroderivat 2-(4-Methylpentyl)-β-tetrahydroanthrachinon (THIHAQ), und (ii) einem Reaktionsträger aus der Reihe 2-Ethylanthrachinon und/oder dem entsprechenden 2-Ethyltetrahydroanthrachinon, wobei die Reaktionsträger gemäß (i) (=IHAQ und THIAHQ) in einer Menge von 5 bis 95 Mol-%, bezogen auf die Summe aller Reaktionsträger, anwesend sind und wobei der Anteil an Tetrahydroanthrachinonen, bezogen auf die Summe aller Reaktionsträger, im Bereich von 5 bis 95 Mol-% liegt.

## Claims

1. Process for the preparation of hydrogen peroxide by the anthraquinone cyclic process, comprising a hydrogenation step, an oxidation step and a step for isolation of the hydrogen peroxide, using a working solution containing at least two differently substituted 2-alkylanthraquinones and/or their 2-alkyltetrahydroanthraquinones,
**characterised in that**
the working solution used contains (i) at least one reaction carrier from the group 2-(4-methyl-3-pentenyl)-anthraquinone (IHEAQ), 2-(4-methylpentyl)-anthraquinone (IHAQ) and their di- and tetrahydroanthraquinone derivatives hydrogenated at the nucleus, and (ii) at least one reaction carrier from the group 2-ethylanthraquinone (EAQ) and/or 2-ethyltetrahydroanthraquinone (THEAQ), wherein the reaction carriers according to (i) are present in an amount of from 5 to 95 mol.%, based on the sum of all the reaction carriers.

2. Process according to claim 1,
**characterised in that**
the working solution contains IHAQ and/or IHEAQ and/or their tetrahydroanthraquinone derivatives hydrogenated at the nucleus, especially β-THIHAQ, as the reaction carrier according to (i).

3. Process according to claim 1 or 2,
**characterised in that**
the working solution used contains reaction carrier according to (i) in an amount of from 10 to 90 mol.%, based on the sum of the reaction carriers.

4. Process according to claim 3,
**characterised in that**
the working solution contains reaction carrier according to (i) in an amount of from 20 to 50 mol.%, based on the sum of the reaction carriers.

5. Process according to any one of claims 1 to 4,
**characterised in that**
the working solution used has been obtained by stocking up a working solution containing at least one reaction carrier according to (ii), especially EAQ and THEAQ, with 2-(4-methyl-3-pentenyl)-anthraquinone (IHEAQ), 2-(4-methylpentyl)-anthraquinone (IHAQ), their tetrahydro derivatives or mixtures of those reaction carriers, during the cyclic process.

6. Process according to any one of claims 1 to 5,
**characterised in that**
there is used during the cyclic process a working solution which contains from 95 to 5 %, preferably from 60 to 20 %, of the reaction carriers in the anthraquinone form and from 5 to 95 %, preferably from 40 to 80 %, of the reaction carriers in the tetrahydroanthraquinone form.

7. Process according to any one of claims 1 to 6,
**characterised in that**
a suspension noble metal catalyst, especially palladium black, or a suspension noble metal catalyst bonded to a support is used as the hydrogenation catalyst in the hydrogenation step.

8. Working solution for the preparation of hydrogen peroxide, **characterised by** a content of (i) 2-(4-methylpentyl)-anthraquinone (IHAQ) and its tetrahydro derivative 2-(4-methylpentyl)-β-tetrahydroanthraquinone (THIHAQ) and (ii) a reaction carrier from the group 2-ethylanthraquinone and/or the corresponding 2-ethyltetrahydroanthraquinone, wherein the reaction carriers according to (i) (= IHAQ and THIAHQ) are present in an amount of from 5 to 95 mol.%, based on the sum of all the reaction carriers, and wherein the proportion of tetrahydroanthraquinones, based on the sum of all the reaction carriers, is in the range from 5 to 95 mol.%.

## Revendications

1. Procédé de fabrication de peroxyde d'hydrogène d'après le processus cyclique anthraquinone, comprenant une étape d'hydrogénation, une étape d'oxydation et une étape permettant l'isolation du peroxyde d'hydrogène, en utilisant une solution de travail contenant au moins deux 2-alkylanthraquinones, différemment substituées, et/ou leurs 2-alkyltétrahydroanthraquinones,
**caractérisé en ce qu'**
on utilise une solution de travail qui contient (i) au moins un support de réaction de la série 2-(4-méthyl-3-pentényle)-anthraquinone (IHEAQ), 2-(4-méthylpentyl)-anthraquinone (IHAQ) et leurs dérivés à hydrogénation nucléaire de di-et tétrahydroanthraquinone, et (ii) au moins un support de réaction de la série 2-éthylanthraquinone (EAQ) et/ou 2-éthyltétrahydroanthraquinone (THEAQ), les supports de réaction selon (i) étant présents dans une quantité de 5 à 95 % en moles, par rapport à la somme de tous les supports de réaction.

2. Procédé selon la revendication 1,
**caractérisé en ce que**
comme support de réaction selon (i), la solution de travail contient, de l'IHAQ et/ou de l'IHEAQ et/ou leurs dérivés à hydrogénation nucléaire de tétrahydroanthraquinone, en particulier de la β-THIHAQ.

3. Procédé selon la revendication 1 ou 2,
**caractérisé en ce qu'**
on utilise une solution de travail qui contient des supports de réaction selon (i) dans une quantité de 10 à 90 % en moles, par rapport à la somme de supports de réaction.

4. Procédé selon la revendication 3,
**caractérisé en ce que**
la solution de travail contient des supports de réaction selon (i) dans une quantité de 20 à 50 % en moles, par rapport à la somme de supports de réaction.

5. Procédé selon l'une quelconque des revendications 1 à 4,
**caractérisé en ce qu'**
on utilise une solution de travail qui a été obtenue par amélioration d'une solution de travail contenant au moins un support de réaction selon (ii), en particulier de l'EAQ et de la THEAQ, pendant le processus cyclique avec de la 2-(4-méthyl-3-pentényl)-anthraquinone (IHEAQ), de la 2-(4-méthylpentyl)-anthraquinone (IHAQ), leurs dérivés tétrahydro ou des mélanges de ces supports de réaction.

6. Procédé selon l'une quelconque des revendications 1 à 5,
**caractérisé en ce qu'**
on utilise, pendant le processus cyclique, une solution de travail qui contient les supports de réaction à raison de 95 à 5 %, de préférence de 60 à 20 %, dans la forme anthraquinone et à raison de 5 à 95 %, de préférence de 40 à 80 %, dans la forme tétrahydroanthraquinone.

7. Procédé selon l'une quelconque des revendications 1 à 6,
**caractérisé en ce qu'**
à l'étape d'hydrogénation, comme catalyseur d'hydrogénation, on utilise, un catalyseur de suspension en métal précieux, en particulier un Mohr de palladium, ou un catalyseur de suspension en métal précieux lié au support.

8. Solution de travail permettant la fabrication de peroxyde d'hydrogène,
**caractérisée par**
une teneur en (i) 2-(4-méthylpentyl)-anthraquinone (IHAQ) et son dérivé tétrahydro 2-(4-méthypentyl)-β-tétrahydroanthraquinone (THIHAQ) et (ii) en un support de réaction de la série 2-éthylanthraquinone et/ou 2-éthyl-tétrahydroanthraquinone correspondante, les supports de réaction selon (i) (=IHAQ et THIAHQ) étant présents dans une quantité de 5 à 95 % en moles, par rapport à la somme de tous les supports de réaction, et la fraction en tétrahydroanthraquinones, par rapport à la somme de tous les supports de réaction, étant de l'ordre de 5 à 95 % en moles.
